# EUROPEAN PATENT APPLICATION

(11) **EP 4 644 428 A1**
(43) Date of publication of application: **05.11.2025**
(21) Application number: 23910917.6
(22) Date of filing: 29.12.2023
(51) Int. Cl.: C08B 37/08, C07C 65/10, A61K 31/728, A61K 31/60, A61K 31/616, A61Q 19/00, A61K 47/36

(54) **HYALURONIC ACID DERIVATIVE, PREPARATION METHOD THEREFOR AND USE THEREOF**

(30) Priority: 30.12.2022 CN 202211726289
(71) Applicant: Bloomage Biotechnology Corporation Limited, Jinan, Shandong 250101 (CN); Dongying First Biochem Industrial Co., Ltd., Dongying, Shandong 257100 (CN)
(72) Inventor: WANG, Chengshan, Jinan, Shandong 250101 (CN); WEI, Jian, Jinan, Shandong 250101 (CN); WEN, Liangliang, Jinan, Shandong 250101 (CN); GUO, Xueping, Jinan, Shandong 250101 (CN); LIU, Zhe, Jinan, Shandong 250101 (CN)
(74) Representative: Abel & Imray LLP
(86) International application number: PCT/CN2023/143151
(87) International publication number: WO 2024/141003

(57) **Abstract**

The present application discloses a hyaluronic acid derivative, which is specifically a hyaluronic acid ester, and is an ester synthesized from hyaluronic acid or salts thereof and salicylate. The hyaluronic acid ester disclosed by the present application has the characteristics of good water solubility, low irritation and mild property, and is capable of optimizing the stratum corneum. Moreover, the water-soluble ester reduces the penetration of salicylic acid in the stratum corneum, so that the action time of salicylic acid in the stratum corneum is increased. The unique chemical structure comprising the salicylic acid and the hyaluronic acid ester of the present application can lock acidic functional groups, thereby reducing the irritation caused by the salicylic acid. The added hyaluronic acid polysaccharide can effectively slow down the penetration of the salicylic acid to the deeper layer of the skin, prolong the action time of the salicylic acid on the epidermis, target the stratum corneum, play a role in long-term regulation of keratinocytes, improve the solubility of the salicylic acid in water phase, and facilitate the use of the formula.

## Description

### FIELD

The present application relates to the field of biotechnology, and in particular to a hyaluronic acid derivative and a preparation method and use thereof.

### BACKGROUND

Hyaluronic acid is a mucopolysaccharide composed of alternating disaccharide units of glucuronic acid and N-acetylglucosamine. Due to its unique molecular structure and physical and chemical properties, it has been successfully used in cosmetics, medical cosmetology, ophthalmic surgery, arthritis treatment, etc.

Hyaluronic acid is widely distributed in nature. In the body, more than 50% of hyaluronic acid is found in the skin, lungs and intestines. In addition, it also exists in the interstitial tissues such as synovial fluid, cartilage, umbilical cord, and blood vessel walls. In early studies, the main source of hyaluronic acid was the umbilical cord. At present, hyaluronic acid products can be extracted from animal tissues such as cockscombs, vitreous humor, brain cartilage, and joint fluid, and can also be obtained by fermentation of bacteria such as *Streptococcus* and *Pseudomonas aeruginosa.* The fermentation method for producing hyaluronic acid is gradually replacing the tissue extraction method due to its low cost, abundant raw materials, easy large-scale production, and high molecular weight of the resulting hyaluronic acid. In recent years, a variety of hyaluronic acid derivatives have been developed through different modifications of hyaluronic acid. In addition to the functions of hyaluronic acid itself, they also have higher stability, biocompatibility and water solubility.

Salicylic acid (SA), also known as o-hydroxybenzoic acid, is a white crystalline powder that can exfoliate the stratum corneum, remove excessively thick stratum corneum, and promote metabolism. However, as a small molecule acid, SA has strong permeability. While dissolving the stratum corneum and destroying the sebum membrane, it can also easily penetrate into the dermis and subcutaneous tissue. Therefore, it does not stay in the stratum corneum for enough time to exert its unique exfoliating effect, and it is more irritating to the microvessels and nerves distributed in the deep skin. The above reasons limit its application.

### SUMMARY

In response to the above issues, the present application provides a derivative of hyaluronic acid and its preparation method. Specifically, the present application adopts the following technical solution:
1. A salicyl hyaluronate, the structural formula of which is set forth in formula (I), 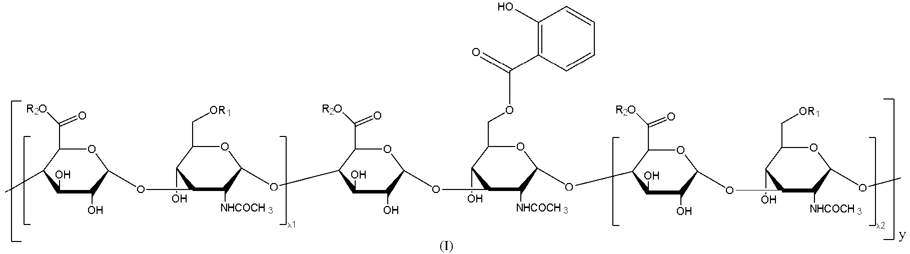 wherein, R₁ is H or 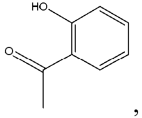 R₂ is H or metal ion, x₁ ≥0, x₂ ≥0, and y≥1.
2. The salicyl hyaluronate according to item 1, the degree of substitution of salicylic acid on the hyaluronic acid or a salt thereof is 15%-60%, preferably the degree of substitution is 25%-50%.
3. The salicyl hyaluronate according to any one of items 1 to 3, the molecular weight of the salicyl hyaluronate is less than or equal to 500 KDa, preferably 5KDa-300KDa, further preferably 5KDa-100KDa.
4. A method for preparing salicyl hyaluronate, the salicyl hyaluronate is an ester synthesized from hyaluronic acid or a salt thereof and salicylate.
5. The method for preparing salicyl hyaluronate according to item 4, the salicyl hyaluronate is obtained by reacting quaternary ammonium hyaluronate with salicylate in the presence of an inorganic base.
6. The method for preparing salicyl hyaluronate according to item 4 or 5, the molar ratio of the quaternary ammonium hyaluronate to the salicylate is 1:(1-10).
7. The method for preparing salicyl hyaluronate according to any one of items 4 to 6, the molar ratio of the inorganic base to the quaternary ammonium hyaluronate is 1:(2-10).
8. The method for preparing salicyl hyaluronate according to any one of items 4 to 7, the inorganic base comprises one or more selected from the group consisting of sodium hydroxide, potassium hydroxide, ammonium hydroxide, barium hydroxide, sodium carbonate, sodium bicarbonate, potassium carbonate and potassium bicarbonate.
9. The method for preparing salicyl hyaluronate according to any one of items 4 to 8, the reaction temperature is 50-100°C, preferably 60-80°C.
10. The method for preparing salicyl hyaluronate according to any one of items 4 to 9, the inorganic base is subjected to a treatment, and the treatment comprises:
   mixing the inorganic base and tetrabutylammonium salt to dissolve them completely and then freeze-drying.
11. The method for preparing salicyl hyaluronate according to any one of items 4 to 10, in the treatment with the inorganic base, the molar ratio of the inorganic base to the tetrabutylammonium salt is 1:(0.1-1).
12. Use of the salicyl hyaluronate according to any one of items 1 to 3 or the salicyl hyaluronate prepared by the method for preparing salicyl hyaluronate according to any one of items 4 to 11 in a skin care product.
13. Use of the salicyl hyaluronate according to any one of items 1 to 3 or the salicyl hyaluronate prepared by the method for preparing salicyl hyaluronate according to any one of items 4 to 11 in the preparation of a exfoliating and/or low-irritation topical product.
14. A composition, comprising the salicyl hyaluronate according to any one of items 1 to 3 or the salicyl hyaluronate prepared by the method for preparing salicyl hyaluronate according to any one of items 4 to 11.

### Effects of the Invention

1. The salicyl hyaluronate of the present application has the characteristics of good water solubility, low irritation, mild nature, and optimizing the stratum corneum. In addition, the water-soluble ester reduces the penetration of salicylic acid in the stratum corneum and increases the duration of action of salicylic acid in the stratum corneum.
2. The unique chemical structure of the salicyl hyaluronate of the present application can lock the acidic functional groups and reduce the irritation of salicylic acid. The increased hyaluronic acid polysaccharide can effectively slow down the penetration of salicylic acid into the deep layers of the skin, prolong the action time of the epidermis, target the stratum corneum, and play a role in long-term regulation of keratinocytes. It also improves the solubility of salicylic acid itself in the aqueous phase, making it easier to use the formula.
3. In the preparation method of salicyl hyaluronate of the present application, the inorganic base is treated by mixed freeze-drying, which improves the catalytic effect of the inorganic base in the reaction solvent, reduces the reaction temperature, and improves the reaction efficiency. By comparison, its catalytic effect is much higher than that of the untreated inorganic base and the organic base. Moreover, the preparation process of the present application is simple and easy to operate, suitable for industrial production, and has broad prospects in the cosmetic raw material market.

### BRIEF DESCRIPTION OF THE DRAWINGS

The accompanying drawings are used to better understand the present application and do not constitute an improper limitation on the present application. wherein:
Figure 1 is a ¹HNMR spectrum of the salicyl hyaluronate prepared in Example 18;
Figure 2 is a full-wavelength ultraviolet scanning spectrum of the salicyl hyaluronate prepared in Example 18.

### DETAIL DESCRIPTION

The following describes exemplary embodiments of the present application, including various details of the embodiments of the present application to facilitate understanding, which should be considered as merely exemplary. Therefore, those of ordinary skill in the art should recognize that various changes and modifications can be made to the embodiments described herein without departing from the scope and spirit of the present application. Also, in the following description, descriptions of well-known functions and structures are omitted for clarity and conciseness.

The present application provides a hyaluronic acid derivative, specifically a salicyl hyaluronate, which is synthesized from hyaluronic acid or a salt thereof and salicylate.

The hyaluronic acid described in the present application refers to a biopolymer material composed of linearly connected repeating units of N-acetyl-D-glucosamine and D-glucuronic acid. The hyaluronic acid or a salt thereof includes hyaluronic acid itself, its salt or a combination thereof. Examples of hyaluronate salts include, but are not limited to, inorganic salts such as sodium hyaluronate, potassium hyaluronate, calcium hyaluronate, magnesium hyaluronate, zinc hyaluronate, gold hyaluronate, cobalt hyaluronate, and organic salts such as quaternary ammonium hyaluronate. In the present application, hyaluronic acid itself or a salt thereof may be used alone, or a combination of two or more hyaluronic acids or salts thereof may be used.

The hyaluronic acid or a salt thereof described in the present application is not limited. In a preferred embodiment, the hyaluronic acid salt is a water-soluble salt of hyaluronic acid, and more preferably quaternary ammonium hyaluronate.

The salicylate described in the present application is preferably an alkyl ester or an alkenyl ester, wherein the alkyl or alkenyl is C1-6, including but not limited to pentyl salicylate, ethyl salicylate, cis-hexene-3-salicylate, hexyl salicylate, isobutyl salicylate, and methyl salicylate.

In a preferred embodiment, the alkyl salicylate is methyl salicylate, i.e., methyl o-hydroxybenzoate, also known as methyl salicylate, which is an organic compound with a chemical formula of C₈H₈O₃, a colorless to light yellow transparent liquid, and has a strong wintergreen oil aroma.

The present application also provides a salicyl hyaluronate, the structural formula of which is set forth in formula (I), 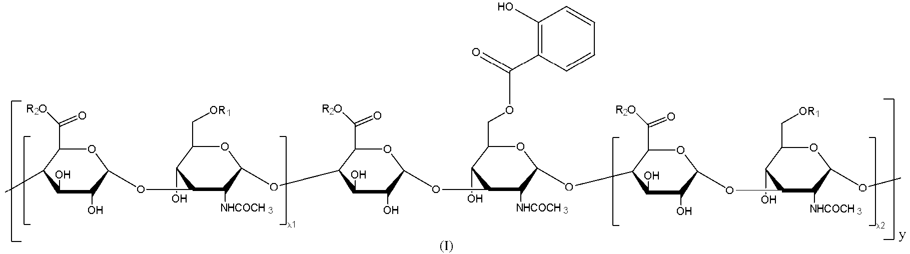 wherein, R₁ is H or 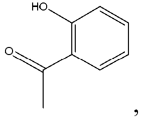 R₂ is H or metal ion, x₁ ≥0, x₂ ≥0, and y≥1; x1, x2 and y are all independent repeating units.

The term "grafting" refers to the reaction of combining appropriate branch chains or functional side groups on the macromolecular chain through chemical bonds, and the resulting product is called a graft copolymer. The grafting rate generally refers to the grafting efficiency, which is grafting rate = [the amount of grafted monomers or copolymer branches grafted to the grafted copolymer/(the total amount of the initially input monomers to be grafted or polymers to be grafted)]*100%. In the present application, the concept of substitution degree is used to express the grafting efficiency. The term "substitution degree" refers to the amount of substance that the active hydroxyl group is substituted on each D-glucose unit of cellulose. In the present application, the substitution degree specifically refers to: [the amount of grafted monomers or copolymer branches grafted to the grafted copolymer/(the total amount of the initially input monomers to be grafted or polymers to be grafted)]*100%.

The degree of substitution of salicyl hyaluronate in the present application is 15%-60%, for example, it can be 16%, 17%, 18%, 19%, 20%, 25%, 30%, 35%, 40%, 45%, 50%, 51%, 52%, 53%, 54%, 55%, 56%, 57%, 58%, or 59%. Preferably, the degree of substitution is 25%-50%. The molecular weight of the salicyl hyaluronate of the present application is less than or equal to 500KDa, for example, it can be 1KDa, 2KDa, 3KDa, 4KDa, 5KDa, 6KDa, 7KDa, 8KDa, 9KDa, 10KDa, 20KDa, 30KDa, 40KDa, 50KDa, 60KDa, 70KDa, 80KDa, 90KDa, 100KDa, 200KDa, 300KDa, 400KDa, or 500KDa. It is preferably 5 KDa-300 KDa, and more preferably 5 KDa-100 KDa.

The present application further provides a method for preparing the hyaluronic acid ester described in the present application, comprising: using an inorganic base as a catalyst and adopting salicylate to carry out a grafting reaction on a quaternary ammonium hyaluronate, that is, grafting methyl salicylate to the quaternary ammonium hyaluronate through a covalent bond to prepare the hyaluronic acid ester.

The quaternary ammonium hyaluronate refers to a hyaluronic acid grafted polymer formed by connecting a positively charged quaternary ammonium group to an active group of hyaluronic acid. After being connected to the quaternary ammonium salt group, hyaluronic acid can enhance its excellent moisturizing properties and improve the adhesion of hyaluronic acid on the surface of skin and hair, thereby enhancing the moisturizing, lubricating, nourishing and repairing effects of hyaluronic acid. Patent documents such as CN101316864 (Shiseido, Japan), CN101715457A (Kewpie, Japan), CN107739417A (YANGZHOU JOYVO WEIKEM Biology Co., Ltd.), and CN107556402A (Chongqing Technology and Business University) have disclosed methods for preparing quaternary ammonium hyaluronate. The quaternary ammonium hyaluronate used in the preparation method of the present application may be quaternary ammonium hyaluronate prepared by any method in the prior art including the above-mentioned patent documents, or may be any quaternary ammonium hyaluronate that can be conventionally purchased. In a preferred embodiment, the present application uses quaternary ammonium hyaluronate prepared by the method disclosed in patent document CN107459590B.

The type of the inorganic base is not limited, and it can be any substance in the art that can promote the reaction in the preparation method of the present application. In the present application, the inorganic base is a catalyst in the reaction process, but the term catalyst does not constitute a limitation on the inorganic base. Those skilled in the art can know that the catalysis refers to the process of causing the rate of a chemical reaction to change. Any substance that can play such a role in changing can be called a catalyst and can also be used in the reaction process of the present application. In some preferred embodiments, the inorganic base can include one or more selected from the group consisting of sodium hydroxide, potassium hydroxide, ammonium hydroxide, barium hydroxide, sodium carbonate, sodium bicarbonate, potassium carbonate and potassium bicarbonate. For example, the inorganic base may be sodium hydroxide; the inorganic base may be potassium hydroxide; the inorganic base may be ammonium hydroxide; the inorganic base may be barium hydroxide; the inorganic base may be sodium carbonate; the inorganic base may be potassium carbonate; the inorganic base may be potassium bicarbonate.

In the preparation method of the present application, a specific embodiment is to first dissolve the quaternary ammonium hyaluronate in an organic solvent, then add methyl salicylate to the organic solvent in which the quaternary ammonium hyaluronate is dissolved, and then add an inorganic base to form a reaction solution, heat and stir, add a salt solution after a certain reaction time and continue stirring, and then perform alcohol precipitation, alcohol washing, dehydration, suction filtration, and drying to obtain the hyaluronic acid ester.

In the above preparation method, the organic solvent used to dissolve the quaternary ammonium hyaluronate is not limited. In a preferred embodiment, the organic solvent is DMSO (dimethyl sulfoxide) or DMF (dimethylformamide).

The heating can be any one of the methods in the art, such as water bath heating, oil bath heating, air bath heating, sand bath heating, electric heating, etc. The heating temperature, that is, the reaction temperature of the quaternary ammonium hyaluronate and methyl salicylate in the preparation method of the present application is not limited, as long as the two can be covalently reacted. In a preferred embodiment, the reaction temperature is 50-100°C, for example, it can be 55°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 85°C, 90°C, 95°C. In a preferred embodiment, the reaction temperature is 60-80°C.

The stirring may be performed by any one of the methods known in the art, as long as the reactants can be evenly mixed and heated.

The certain reaction time refers to the time required for the quaternary ammonium hyaluronate and methyl salicylate to react completely, and there is no limitation as long as the two can react completely.

The salt solution is not limited, for example, it can be a sodium chloride solution, a potassium chloride solution, a calcium chloride solution, etc. During the reaction, in a preferred embodiment, the salt solution is a NaCl solution, the concentration of the salt solution is 2%-5%, and the volume is half or more of the volume of the reaction solution.

In the above preparation method, after adding the salt solution, stirring is continued for a certain period of time, for example, 10-50 minutes, to help precipitation and complete ion replacement, and then an alcohol solution can be used for precipitation and washing. The concentration of the alcohol solution is not limited, preferably 60%-90%, and the number of washing times is not limited, preferably 1-10 times. In a preferred preparation method, after washing with alcohol, the mixture is dehydrated once with ethanol, and then filtered and vacuum dried to obtain the final product.

In the above preparation method, the molar ratio of the quaternary ammonium hyaluronate to methyl salicylate is not limited. In a preferred embodiment, the molar ratio of the quaternary ammonium hyaluronate to methyl salicylate is 1:(1-10), for example, it can be 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5.

In the above preparation method, the molar ratio of the inorganic base to the quaternary ammonium hyaluronate is not limited. In a preferred embodiment, the molar ratio of the inorganic base to the quaternary ammonium hyaluronate is 1:(2-10), for example, it can be 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5.

In a preferred embodiment, the inorganic base is a treated inorganic base.

The treatment method includes mixing and dissolving the inorganic base and tetrabutylammonium salt and then freeze-drying.

The mixing can be carried out by any one of the conventional methods in the art, and the dissolving is to mix and dissolve the inorganic base and the tetrabutylammonium salt in water.

The type of the tetrabutylammonium salt is not limited, and may be, for example, tetrabutylammonium fluoride, tetrabutylammonium chloride, tetrabutylammonium bromide or tetrabutylammonium iodide.

Freeze drying is freeze-drying, which is a drying method that freezes the water-containing material into a solid and uses the sublimation property of water under low temperature and low pressure conditions to dehydrate the material at low temperature to achieve drying. The freeze-drying process of the present application can be performed by any method in the art, for example, pre-freezing with a refrigerator or a freeze dryer or liquid nitrogen, and then freeze-drying.

In the above treatment process, the molar ratio of the inorganic base to the tetrabutylammonium salt is not limited. In a preferred embodiment, the molar ratio of the inorganic base to the tetrabutylammonium salt is 1:0.1-1:1, for example, it can be 1:0.2, 1:0.25, 1:0.3, 1:0.35, 1:0.4, 1:0.45, 1:0.5, 1:0.55, 1:0.6, 1:0.65, 1:0.7, 1:0.75, 1:0.8, 1:0.85, 1:0.9, 1:0.95.

The present application further provides use of any one of the hyaluronic acid esters described above or the hyaluronic acid esters prepared using the preparation method provided by the present application in skin care products or skin care items.

The skin care products or skin care items include but are not limited to water, lotion, ointment, cream, essence, sunscreen, oil, makeup remover, solution, gel, shower gel, shampoo, facial cleanser, shampoo cream, shaving cream, shaving foam, shaving water, moisturizing lotion, moisturizing spray, moisturizing mask, toner, freshener, softener, exfoliating gel, acne gel, facial cream, body cream, hand cream, foot cream, repair essence, moisturizing lotion, moisturizing cream, repair lotion, moisturizing lipstick, lip gloss, lip glaze, depilatory cream, etc.

In a preferred embodiment, the skin care product is a skin care product with an exfoliating function.

The hyaluronic acid ester provided in the present application as a new hyaluronic acid derivative can be completely soluble in water and has good water solubility. It can be prepared into a cosmetic raw material, and can be prepared into cosmetics by mixing with various cosmetic raw materials. In addition, the molecular weight of the hyaluronic acid ester can be selected according to different needs, providing more possibilities for the cosmetic raw material market.

The hyaluronic acid ester of the present application has the characteristics of both hyaluronic acid and salicylic acid, and the unique chemical structure can lock the acidic functional groups and reduce the irritation of salicylic acid. The added hyaluronic acid polysaccharide can effectively slow down the penetration of salicylic acid into the deep layers of the skin, prolong the action time of the epidermis, target the stratum corneum, and have the characteristics of low irritation, mild nature, and optimization of the stratum corneum. Through human skin tests, it can be clearly seen that the hyaluronic acid ester of the present application has lower irritation and a good keratin exfoliation rate compared with the composition of salicylic acid and hyaluronic acid ester.

The present application provides a method for preparing the salicyl hyaluronate, and optimizes the types and amounts of raw materials in the preparation method. The inorganic base is treated by mixed freeze-drying, thereby improving the catalytic effect of the inorganic base in the reaction solvent, reducing the reaction temperature, and improving the reaction efficiency. By comparison, its catalytic effect is much higher than that of the untreated inorganic base and the organic base. At the same time, hyaluronic acid ester with a higher degree of substitution can also be prepared by optimizing the preparation conditions.

### EXAMPLES

### Example 1

1) Treatment of the inorganic base: 5 mmol potassium bicarbonate and 2.5 mmol tetrabutylammonium chloride were mixed and dissolved in 10 ml purified water. After being completely dissolved, they were pre-frozen with liquid nitrogen and freeze-dried to obtain the treated inorganic base.
2) Preparation of quaternary ammonium hyaluronate: hyaluronic acid with a molecular weight of 100 KDa was selected, and quaternary ammonium hyaluronate was prepared by the method disclosed in Chinese patent CN107459590B.
3) Preparation of water-soluble hyaluronic acid ester: 10 mmol of the quaternary ammonium hyaluronate prepared in step 2) was dissolved in DMSO (dimethyl sulfoxide), and after it was completely dissolved, 50 mmol of methyl salicylate was added;
   all the inorganic base prepared in step 1) was added, stirred and reacted at 80°C for 4h;
   half of the total volume of 2% NaCl solution was added, and stirred for 20 minutes, and then ethanol was added to precipitate. The mixture was left to stand, and the supernatant was taken and washed with 80% ethanol. The process was repeated 5 times, and the supernatant was dehydrated with ethanol once, filtered and vacuumed dried to obtain water-soluble hyaluronic acid ester powder.

The degree of substitution of the product was detected by NMR quantitative analysis, which was achieved by comparing different absorption peak intensities. When performing quantitative analysis, the integral of the absorption peak intensity of the H atom was proportional to its molar concentration, and then the degree of substitution was calculated. The degree of substitution was 15%.

### Examples 2-27

Water-soluble hyaluronic acid ester powder was prepared according to the method of Example 1, wherein the types and amounts of raw materials used were set forth in Table 2, and other parts not mentioned were the same as those in Example 1.

The structural formula of the water-soluble hyaluronic acid ester prepared in Example 18 was as follows:

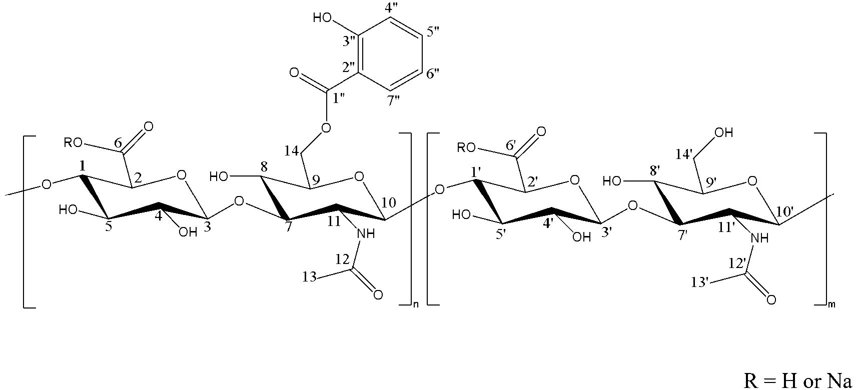

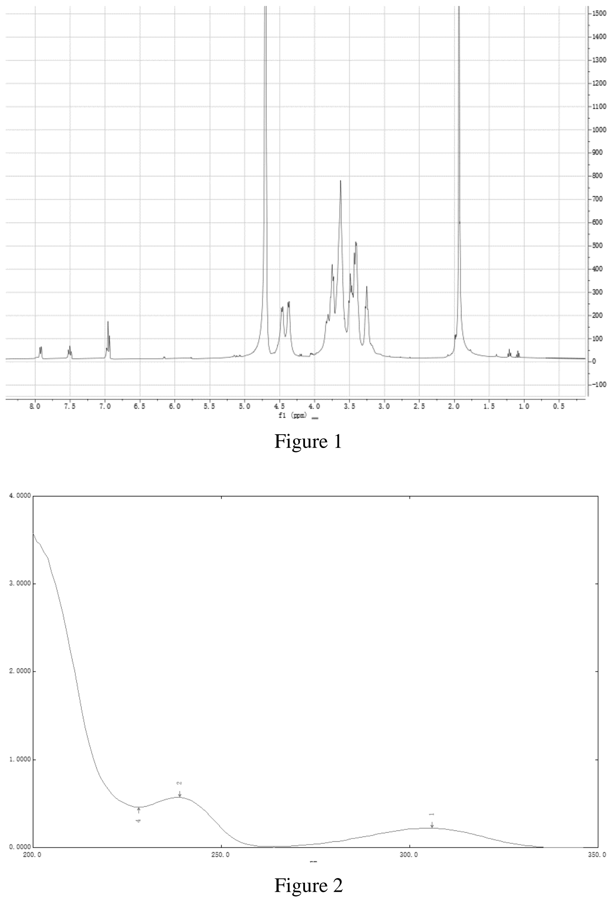

The ¹HNMR spectrum was set forth in Figure 1.

**Table 1. ¹H-NMR data of sodium salicyl hyaluronate in D₂O.**

| Number | Chemical shift δ | Multiplicity | Proton number |
|---|---|---|---|
| 7" | 8.015 | d | 1 |
| 5" | 7.600 | t | 1 |
| 6" | 7.053 | m | 1 |
| 4" | 7.032 | m | 1 |
| 10/10' | 4.555 | m | 2 |
| 3/3' | 4.478 | m | 2 |
| 14/14' | 3.903 | m | 4 |
| 11/11' | 3.903 | m | 2 |
| 7/7' | 3.846 | m | 2 |
| 1/1' | 3.846 | m | 2 |
| 9/9' | 3.726 | m | 2 |
| 2/2' | 3.586 | m | 2 |
| *5*/*5'* | 3.568 | m | 2 |
| 8/8' | 3.526 | m | 2 |
| 4/4' | 3.353 | m | 2 |
| 13/13' | 2.030 | s | 6 |

The ¹H spectrum shows 16 groups of hydrogen, and the integral ratios from low field to high field hydrogen are 1:1:1:1:2:2:4:2:2:2:2:2:2:2:2:6, which is consistent with the structure of this product. Among them, the hydrogen at δ8.015 is a group of doublet, with a proton number of 1, which was attributed to the 7" position hydrogen; the hydrogen at δ7.600 was a group of triplet, with a proton number of 1, which is attributed to the 5" position hydrogen; the hydrogen at δ7.053 is a group of multiplet, with a proton number of 1, which is attributed to the 6" position hydrogen; the hydrogen at δ7.032 is a group of multiplet, with a proton number of 1, which is attributed to the 4" position hydrogen. Chemical shifts in the range of δ7-δ8.5 are generally caused by benzene ring structures. The above results indicates that this product contains benzene ring structures and has a 1:1 relationship with the hyaluronic acid molecule.

Ultraviolet absorption spectrum of benzene ring: 203nm (ε=8700) is called the E2 band; there is a series of relatively weak absorption bands at 230-270nm, called the fine structure, centered at 254nm (ε=204), and is called the B band. When groups such as -OCH₃, -CHO, -COOH, and -NO₂ were introduced, both the E2 band and the B band generally produced a red shift. The UV full wavelength scanning spectrum of this product is set forth in Figure 2. The peak in the range of 230nm-240nm is the E2 band of the benzene ring; the peak in the range of 300nm-310nm is the B band of the benzene ring. The above results show that this product contains carbonyl and substituted benzene ring structures.

**Table 2. Raw materials and amounts thereof in the Examples**

| Groups | Treatment of inorganic base | | | | Preparation of quaternary ammonium hyaluronate | Preparation of water-soluble hyaluronic acid ester | | |
|---|---|---|---|---|---|---|---|---|
| | Amount of Inorganic base /mmol | Types of inorganic bases | Tetrabutylammonium chloride dosage /mmol | Processing method | HA molecular weight /kDa | Amount of quaternary ammonium hyaluronate /mmol | Amount of methyl salicylate /mmol | Reaction temperature °C |
| Example 1 | 5 | Potassium Bicarbonate | 2.5 | freeze-drying | 100 | 10 | 50 | 80 |
| Example 2 | 0.8 | Potassium Bicarbonate | 0.4 | freeze-drying | 100 | 10 | 50 | 80 |
| Example 3 | 6 | Potassium Bicarbonate | 3 | freeze-drying | 100 | 10 | 50 | 80 |
| Example 4 | 1 | Potassium Bicarbonate | 0.5 | freeze-drying | 100 | 10 | 120 | 80 |
| Example 5 | 1 | Potassium Bicarbonate | 0.5 | freeze-drying | 100 | 10 | 1 | 80 |
| Example 6 | 1 | Potassium Bicarbonate | 0.5 | freeze-drying | 100 | 10 | 100 | 80 |
| Example 7 | 1 | Potassium Bicarbonate | 0.5 | freeze-drying | 100 | 10 | 10 | 80 |
| Example 8 | 1 | Potassium Bicarbonate | 1.2 | freeze-drying | 100 | 10 | 50 | 80 |
| Example 9 | 1 | Potassium Bicarbonate | 0.08 | freeze-drying | 100 | 10 | 50 | 80 |
| Example 10 | 1 | Potassium Bicarbonate | 1 | freeze-drying | 100 | 10 | 50 | 80 |
| Example 11 | 1 | Potassium Bicarbonate | 0.1 | freeze-drying | 100 | 10 | 50 | 80 |
| Example 12 | 1 | Potassium Bicarbonate | 0.5 | freeze-drying | 2500 | 10 | 50 | 80 |
| Example 13 | 1 | Potassium Bicarbonate | 0.5 | freeze-drying | 1000 | 10 | 50 | 80 |
| Example 14 | 1 | Potassium Bicarbonate | 0.5 | freeze-drying | 9 | 10 | 50 | 80 |
| Example 15 | 1 | Potassium Bicarbonate | 0.5 | freeze-drying | 400 | 10 | 50 | 80 |
| Example 16 | 1 | Potassium Bicarbonate | 0.5 | freeze-drying | 100 | 10 | 50 | 80 |
| Example 17 | 1 | Potassium Bicarbonate | 0.5 | freeze-drying | 100 | 10 | 50 | 60 |
| Example 18 | 1 | Potassium Bicarbonate | 0.5 | freeze-drying | 100 | 10 | 50 | 50 |
| Example 19 | 1 | Potassium Bicarbonate | 0.5 | freeze-drying | 100 | 10 | 50 | 100 |
| Example 20 | 1 | Potassium Bicarbonate | 0.5 | freeze-drying | 100 | 10 | 50 | 40 |
| Example 21 | 1 | Potassium Bicarbonate | 0.5 | freeze-drying | 100 | 10 | 50 | 120 |
| Example 22 | 1 | potassium hydroxide | 0.5 | freeze-drying | 100 | 10 | 50 | 80 |
| Example 23 | 1 | Potassium carbonate | 0.5 | freeze-drying | 100 | 10 | 50 | 80 |
| Example 24 | 1 | 4-Dimethylaminopyridine | 0.5 | freeze-drying | 100 | 10 | 50 | 80 |
| Example 25 | 1 | triethylamine | 0.5 | freeze-drying | 100 | 10 | 50 | 80 |
| Example 26 | 1 | Potassium Bicarbonate | 0.5 | / | 100 | 10 | 50 | 80 |
| Example 27 | 1 | Potassium Bicarbonate | 0.5 | Rotary evaporation vacuum drying | 100 | 10 | 50 | 80 |

The degree of substitution of the prepared hyaluronic acid ester was detected by nuclear magnetic resonance, as set forth in Table 3.

**Table 3. Degree of substitution and molecular weight of hyaluronic acid esters prepared in various Examples.**

| Groups | Degree of substitution | molecular weight of hyaluronic acid ester/kDa |
|---|---|---|
| Example 1 | 15% | 10 |
| Example 2 | 4% | 10 |
| Example 3 | 0.3% | 10 |
| Example 4 | 0.5% | 10 |
| Example 5 | 0.1% | 10 |
| Example 6 | 17% | 10 |
| Example 7 | 15% | 10 |
| Example 8 | 13% | 10 |
| Example 9 | 13% | 10 |
| Example 10 | 60% | 10 |
| Example 11 | 17% | 10 |
| Example 12 | 18% | 500 |
| Example 13 | 45% | 300 |
| Example 14 | 59% | 5 |
| Example 15 | 53% | 60 |
| Example 16 | 57% | 10 |
| Example 17 | 40% | 10 |
| Example 18 | 26% | 10 |
| Example 19 | 19% | 10 |
| Example 20 | 3.0% | 10 |
| Example 21 | 10% | 10 |
| Example 22 | 22% | 10 |
| Example 23 | 33% | 10 |
| Example 24 | 0% | 10 |
| Example 25 | 0% | 10 |
| Example 26 | 4% | 10 |
| Example 27 | 7% | 10 |

### Test Examples

### Test Example 1. Cytotoxicity Test

### 1.1 Sample: salicyl hyaluronate obtained in Example 18.

### 1.2 Experimental methods and procedures:

### 1.2.1 Plating

HaCaT cells in logarithmic growth phase were inoculated in a 96-well plate at a density of 1aC0⁵/mL, 100 µL per well. The culture system was DMEM basal culture medium supplemented with 10% fetal bovine serum. The inoculated cells were placed in a carbon dioxide incubator at 37°C and 5%CO₂ for conventional culture for 24 hours.

### 1.2.2 Sample solution preparation

The samples were made into 2.0% solution with serum-free culture medium, filtered through a 0.22 µm filter membrane for sterilization, and the final concentrations were 0.05%, 0.1%, 0.2%, and 0.5%, which were prepared and used immediately.

### 1.2.3 Dosing

After 24 hours of routine culture, the old culture medium was discarded, and the experimental group was replaced with 100 µL sample, and the normal control group (control) was added with an equal amount of serum-free culture medium, with 6 parallel wells at each level.

### 1.3 Detection

After culturing for 24 hours, CCK-8 was used to detect the relative cell proliferation rate. The culture medium was discarded, and 100 µL of CCK-8 diluted 10 times with serum-free culture medium was added to each well. The cells were placed in a cell culture incubator and incubated for 2 hours. The absorbance was measured at a wavelength of 450 nm using an enzyme-labeled instrument. The relative growth rate (RGR) is the ratio of the absorbance of the experimental group to the absorbance of the normal control group. According to the requirements of GB/T16886.5-2017, when the RGR is lower than 70%, the sample is considered to be cytotoxic.

### 1.4 Results and Analysis:

A relative growth rate of 70% or more is considered non-cytotoxic. Salicyl hyaluronate has no cytotoxicity at concentration of 0.5% and below.

**Table 4. Relative cell growth rate of different concentrations of salicyl hyaluronate**

| Concentration | Control | 0.05% | 0.1% | 0.2% | 0.5% |
|---|---|---|---|---|---|
| Relative Growth rate | 100.00±5.85 | 85.14±2.24*** | 84.41±5.37*** | 78.93±3.89*** | 78.75±3.33*** |

| | | | | | |
|---|---|---|---|---|---|
| Note: *** indicates that compared with Control, ρ<0.001. | | | | | |

### Test Example 2. Irritation Study

### 1.1 Sample:

Sample 1: salicyl hyaluronate obtained in Example 18 was dissolved in water with a mass concentration of 5%.
Sample 2: a composition of hyaluronic acid and salicylic acid was dissolved in water, wherein the molecular weight of the hyaluronic acid was 100 KDa, the mass concentration of the hyaluronic acid was 4.35%, and the mass concentration of the salicylic acid was 0.7%.

### 1.2 Experimental methods and procedures:

Healthy volunteers were recruited to participate in this project voluntarily.

### Exclusion criteria:

① Those with severe systemic diseases, immunodeficiency or autoimmune diseases, skin diseases or disease histories (severe freckles, atopic dermatosis, psoriasis, eczema, severe acne, etc.);
② People who are receiving dermatological treatment or cosmetic surgery, or who have used hormone drugs or immunosuppressants within one month;
③ Those with allergic constitution, allergic dermatitis, or active allergic diseases;
④ People who are pregnant, breastfeeding or menopausal;
⑤ Other skin manifestations that affect the test, and people who are considered unsuitable to participate in the project based on clinical evaluation.

The effective number of subjects: 30.

The subjects cleaned their faces with cleanser, dried them with a tissue, and waited for 15 minutes. 20µL of each sample was dropped onto a circular filter paper with a diameter of 7mm and randomly attached to the nasolabial grooves on both sides of the subjects. The subjects evaluated the discomfort level of itching, tingling, and burning sensation in the test area at 0 minutes, 1 minute, 5 minutes, and 10 minutes. The sum of the sensory scores at each time point was the total sensory score.

Scoring criteria: 0 points: no tingling, itching, or burning sensation; 1 point: mild tingling, itching, or burning sensation (slightly felt); 2 points: moderate tingling, itching, or burning sensation (strong sensation, but within an acceptable range); 3 points: obvious tingling, itching, or burning sensation (relatively strong sensation, unbearable).

### 1.3 Statistical methods:

Paired T test was used for statistical analysis; the test level was α = 0.05

If the test mean was in a beneficial direction and the test result p<0.05, indicating it was effective.

### 1.4 Results and Analysis:

The overall skin irritation score of the sample containing 5% salicyl hyaluronate within 10 minutes after application is lower, proving that salicyl hyaluronate is less irritating.

**Table 5. Subjects' irritation scores of samples 1 and 2.**

| Sample | sensation | 0min | 1min | 5min | 10min |
|---|---|---|---|---|---|
| Sample 1 | itching | 0.07(0,2) | 0.13(0,2) | 0.23(0,2) | 0.20(0,2) |
| | burning | 0.07(0,1) | 0.17(0,2) | 0.17(0,2) | 0.10(0,1) |
| | tingling | 0.10(0,3) | 0.03(0,1) | 0.10(0,1) | 0.13(0,2) |
| | overall score | 0.24±0.02 | 0.33±0.07 | 0.50±0.07 | 0.43±0.05 |
| Sample 2 | itching | 0.07(0,2) | 0.17(0,2) | 0.23(0,2) | 0.23(0,2) |
| | burning | 0.07(0,2) | 0.17(0,2) | 0.2(0,2) | 0.20(0,2) |
| | overall score | 0.10(0,3) | 0.07(0,2) | 0.13(0,2) | 0.17(0,3) |
| | tingling | 0.24±0.07 | 0.41±0.05 | 0.56±0.05 | 0.60±0.05 |

### Test Example 3: Determination of keratinocyte exfoliation ability - stratum corneum desquamation

### 1.1 Sample:

Sample 1: salicyl hyaluronate obtained in Example 18 was dissolved in water with a mass concentration of 5%.
Sample 2: a composition of hyaluronic acid and salicylic acid was dissolved in water, wherein the molecular weight of the hyaluronic acid was 100 KDa, the mass concentration of the hyaluronic acid was 4.35%, and the mass concentration of the salicylic acid was 0.7%.

### 1.2 Experimental methods and procedures:

Twenty-five healthy subjects were recruited, and the flexor side of the forearm was wiped with dry tissue paper, and then a 3 cm × 3 cm test area was marked at the same position on the flexor side of the left and right forearms.

Application of the samples: 20 µL of each sample was applied to the test areas of the left and right arms, massaged for 30 seconds, waiting for one minute, rinsed with warm water for 10 seconds, and the moisture was absorbed with dry tissue.

### 1.3 Desquamation determination method:

15 minutes after absorbing the water, the peeling film was gently pressed on the test area to collect the desquamation, and then the percentage of keratin exfoliation was measured with Visioscope PC 35.

### 1.4 Results and Analysis:

Salicyl hyaluronate with the concentration of 5% has strong exfoliating ability.

**Table 6. Exfoliation rate of sample 1 and sample 2 for keratin.**

| Sample | Sample 1 | Sample 2 |
|---|---|---|
| Exfoliation rate (%) | 12.52±7.32 | 12.68±6.49 |

Although the embodiments of the present application are described above, the present application is not limited to the above-mentioned specific embodiments and application fields. The above-mentioned specific embodiments are merely illustrative and guiding, and not restrictive. Those ordinary skilled in the art may make many forms under the guidance of this specification and without departing from the scope of protection of the claims of this application, all of which are protected by this application.

## Claims

1. A salicyl hyaluronate, the structural formula of which is set forth in formula (I), 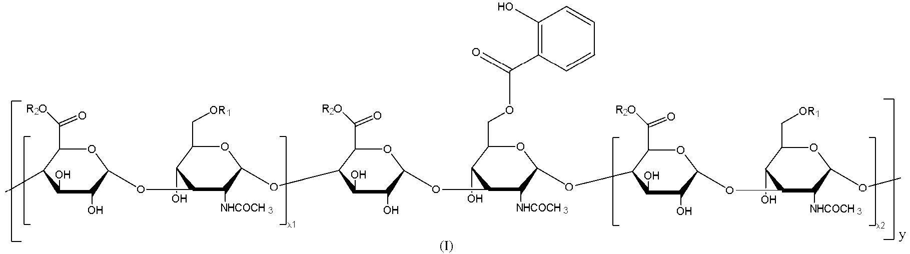 wherein R₁ is H or 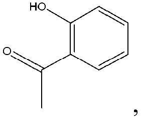 R₂ is H or metal ion, x₁ ≥0, x₂ ≥0, and y≥1.

2. The salicyl hyaluronate according to claim 1, wherein the degree of substitution of salicylic acid on the hyaluronic acid or a salt thereof is 15%- 60%.

3. The salicyl hyaluronate according to claim 2, wherein the degree of substitution is 25%-50%.

4. The salicyl hyaluronate according to any one of claims 1 to 3, wherein the molecular weight of the salicyl hyaluronate is less than or equal to 500 KDa.

5. The salicyl hyaluronate according to claim 4, wherein the molecular weight of the salicyl hyaluronate is 5KDa-300KDa.

6. The salicyl hyaluronate according to claim 4, wherein the molecular weight of the salicyl hyaluronate is 5KDa-100KDa.

7. A method for preparing salicyl hyaluronate, wherein the salicyl hyaluronate is an ester synthesized from hyaluronic acid or a salt thereof and salicylate.

8. The method for preparing salicyl hyaluronate according to claim 7, wherein the salicyl hyaluronate is obtained by reacting quaternary ammonium hyaluronate with salicylate in the presence of an inorganic base.

9. The method for preparing salicyl hyaluronate according to claim 7 or 8, wherein the molar ratio of the quaternary ammonium hyaluronate to the salicylate is 1:(1-10).

10. The method for preparing salicyl hyaluronate according to claim 9, wherein the molar ratio of the inorganic base to the quaternary ammonium hyaluronate is 1:(2-10).

11. The method for preparing salicyl hyaluronate according to claim 7, wherein the inorganic base comprises one or more selected from the group consisting of sodium hydroxide, potassium hydroxide, ammonium hydroxide, barium hydroxide, sodium carbonate, sodium bicarbonate, potassium carbonate and potassium bicarbonate.

12. The method for preparing salicyl hyaluronate according to claim 7, wherein the reaction temperature is 50-100°C.

13. The method for preparing salicyl hyaluronate according to claim 12, wherein the reaction temperature is 60-80°C.

14. The method for preparing salicyl hyaluronate according to claim 7, wherein the inorganic base is subjected to a treatment, and the treatment comprises:
mixing the inorganic base and tetrabutylammonium salt to dissolve them completely and then freeze-drying.

15. The method for preparing salicyl hyaluronate according to claim 14, wherein in the treatment with the inorganic base, the molar ratio of the inorganic base to the tetrabutylammonium salt is 1:(0.1-1).

16. Use of the salicyl hyaluronate according to any one of claims 1 to 6 or the salicyl hyaluronate prepared by the method for preparing salicyl hyaluronate according to any one of claims 7 to 15 in a skin care product.

17. Use of the salicyl hyaluronate according to any one of claims 1 to 6 or the salicyl hyaluronate prepared by the method for preparing salicyl hyaluronate according to any one of claims 7 to 15 in the preparation of a exfoliating and/or low-irritation topical product.

18. A composition, comprising the salicyl hyaluronate according to any one of claims 1 to 6 or the salicyl hyaluronate prepared by the method for preparing salicyl hyaluronate according to any one of claims 7 to 15.
